# EUROPEAN PATENT SPECIFICATION

(11) **EP 3 787 556 B1**
(45) Date of publication and mention of the grant of the patent: **26.02.2025**
(21) Application number: 19772837.1
(22) Date of filing: 10.07.2019
(51) Int. Cl.: A61F 2/16

(54) **INTRAOCULAR LENS INJECTOR**
INTRAOKULARLINSEN-INJEKTOR
INJECTEUR DE LENTILLE INTRAOCULAIRE

(30) Priority: 10.07.2018 US 201862696072 P
(43) Date of publication of application: 10.03.2021
(73) Proprietor: Alcon Inc., 1701 Fribourg (CH)
(72) Inventor: ZACHER, Rudolph F., Trabuco Canyon, California 92679 (US); LIU, Jian, Keller, Texas 76248 (US)
(74) Representative: Murgitroyd & Company
(86) International application number: PCT/IB2019/055887
(87) International publication number: WO 2020/012385

(56) References cited:
- WO-A1-98/12969
- US-A- 5 304 182
- US-A1- 2005 149 056

## Description

### TECHNICAL FIELD

The present disclosure relates to ophthalmic surgery and, more specifically, to intraocular lens (IOL) injector comprising an IOL compressor and a method of compressing an IOL prior to injection of the IOL into an eye.

### BACKGROUND

In ophthalmology, eye surgery, or ophthalmic surgery, saves and improves the vision of tens of thousands of patients every year. However, given the sensitivity of vision to even small changes in the eye and the minute and delicate nature of many eye structures, ophthalmic surgery is difficult to perform and the reduction of even minor or uncommon surgical errors or modest improvements in accuracy of surgical techniques can make an enormous difference in the patient's vision after the surgery.

Light enters the human eye through a clear cornea that is located on the outer part of the eye and covers the pupil and iris. The light travels through the pupil and then encounters the lens, located behind the iris. As the light travels through the lens, the lens refracts the light so that it focuses on the retina, located in the back of the eye. Special cells in the retina detect the light and transmit signals based on the light via the optic nerve to the brain, which interprets the signals as vision.

Vision quality is, therefore, influenced by a number of factors, including the transparency and refractive properties of the cornea and the lens. Unfortunately, as people age or due to trauma or disease, the lens may be become less transparent and a cataract develops. Cataracts cause deterioration of vision and are often surgically corrected. During some cataract surgeries, the lens is surgically removed and replaced with an artificial intraocular lens (IOL).

Many cataractous lenses are removed by a surgical technique called phacoemulsification. During this procedure, an opening is made in the anterior capsule and a phacoemulsification cutting tip is inserted into the diseased lens and vibrated ultrasonically. The vibrating cutting tip liquefies or emulsifies the lens so that the lens may be aspirated out of the eye. The diseased lens, once removed, is replaced by an artificial lens, also referred to as an intraocular lens (IOL).
The IOL is injected into the eye through a small incision, sometimes the same incision used to remove the diseased lens. An IOL injector is used to deliver an IOL into the eye.

The relevant state of the art is represented by US 2005/149056 A1, US 5304182 A and WO 98/12969 A1. US 2005/149056 discloses an injector device for injecting an IOL into an eye, said injector device comprising: an injector body having a longitudinal passageway and an opening formed in a side wall of said body for placement of an IOL therein; a compressor having a leading surface, a finger push surface, a top wall, a bottom wall and opposite side walls, said compressor adapted to be received in said opening and movable between opened and closed positions with respect to said injector body; and a locking mechanism to prevent said compressor from moving.

### SUMMARY

The scope of the invention is in accordance with the appended claims.

The present invention provides an intraocular lens injector as detailed in claim 1. Also provided is a method according to claim 5. Advantageous features are provided in the dependent claims.

An aspect of the present disclosure relates to an intraocular lens injector as defined in claim 1. The intraocular lens injector includes an injector body defining a bore; a nozzle coupled to the injector body, the nozzle including an opening in fluid communication with the bore; a plunger receivable into the bore and moveable therein; and an IOL compressor coupled to the injector body. The IOL compressor includes a housing; a compression chamber formed within the housing and adapted to receive an IOL; and a transversely depressible button extending through a slot formed in the housing, the transversely depressible button moveable within the slot. The transversely depressible button includes a pad located outside of the housing of the compression chamber; an internal portion located inside of the compression chamber, the internal portion having a compression surface adapted to compress the IOL disposed in the compression chamber; and a stem connecting the pad and the internal portion. The stem is slideably disposed within the slot.

Another aspect is directed to a method of compressing an intraocular lens according to claim 5. The method includes applying a transverse compression force to an intraocular lens comprising a base, a trailing haptic coupled to the base, and a leading haptic coupled to the base; positioning a trailing haptic of the intraocular lens into a tucked configuration as the intraocular lens is compressed; restraining longitudinal displacement of the leading haptic as the intraocular lens is compressed; positioning the leading haptic into a tucked configuration as the compressed intraocular lens is longitudinally displaced; and maintaining both the leading haptic and the trailing haptic in a tucked configuration as the compressed intraocular lens continues to be longitudinally displaced.

The various aspects may include one or more of the following features. The housing of the compressor may include an exterior surface that defines a seat. The pad of the transversely depressible button may include a seating surface, and the seating surface may be adapted to contact the seat to stop movement of the transversely depressible button in a first trailing haptic in a tucked configuration as the compressed intraocular lens continues to be longitudinally displaced.

The various aspects may include one or more of the following features. The housing of the compressor may include an exterior surface that defines a seat. The pad of the transversely depressible button may include a seating surface, and the seating surface may be adapted to contact the seat to stop movement of the transversely depressible button in a first transverse direction. According to the invention, at least one flexible wing extends from the stem. The at least one wing is adapted to flex inwardly when passing through the slot and to expand outwardly when the at least one flexible wing is received into the compression chamber. The compression chamber defines an interior surface. The at least one flexible wing cooperates with the interior surface to form a lock that is adapted to hold the transversely depressible button into a depressed state when the at least one flexible wing is received into the compression chamber. The compression chamber may define a slot adapted to receive a leading haptic of the IOL and to restrain longitudinal movement of the leading haptic as the IOL is compressed within the compression chamber. The compression chamber may include and interior surface that includes a curved portion adapted to guide a trailing haptic of the IOL into a tucked configuration as the IOL is compressed within the compression chamber.

The various aspects may also include one or more of the following features. The compressed intraocular lens may be delivered from an intraocular lens injector. Applying a transverse compression force to an intraocular lens may include depressing a transversely depressible button to compress the intraocular lens between the button and an interior surface of an intraocular lens compressor. Positioning a trailing haptic of the intraocular lens into a tucked configuration as the intraocular lens is compressed may include engaging a tip of the trailing haptic with a curved surface to guide the trailing haptic into an abutting and conforming relationship with a base of the intraocular lens. Restraining longitudinal displacement of the leading haptic as the intraocular lens is compressed may include positioning the leading haptic in a slot that is adapted to define a path of travel of the leading haptic as the intraocular lens is compressed. Positioning the leading haptic into a tucked configuration as the compressed intraocular lens is longitudinally displaced may include longitudinally displacing the compressed intraocular lens while restraining longitudinal movement of the leading haptic such that the leading haptic abuts and conforms to the a shape of the base

### BRIEF DESCRIPTION OF THE DRAWINGS

For a more complete understanding of the present disclosure and the associated features and advantages, reference is now made to the following description, taken in conjunction with the accompanying drawings, which are not to scale, and in which:
FIG. 1 is a perspective view of an example IOL injector.
FIG. 2 is a longitudinal cross-sectional view of the exemplary IOL injector of FIG. 1.
FIG. 3 shows an exemplary one-piece IOL.
FIG. 4 shows an exemplary two-piece IOL including a base and an optic.
FIG. 5 is detail view of an example IOL compressor included in an IOL injector.
FIG. 6 is a perspective view of an exemplary IOL injector showing detail of an example IOL compressor.
FIG. 7 is another detail view of the IOL compressor in which a button operable to compress an IOL or part thereof is shown in a fully actuated position.
FIGs. 8A and 8B show an IOL device disposed in an IOL compressor in uncompressed configuration and compressed configuration, respectively.
FIG. 9 shows an IOL device in a compressed configuration with a leading haptic conformed to a perimeter of a base of the IOL device.
FIG. 10 is an example method for compressing an IOL or component thereof.

### DETAILED DESCRIPTION

In the following description, details are set forth by way of example to facilitate discussion of the disclosed subject matter. It should be apparent to a person of ordinary skill in the art, however, that the disclosed implementations are exemplary and not exhaustive of all possible implementations.

For the purposes of promoting an understanding of the principles of the present disclosure, reference will now be made to the implementations illustrated in the drawings, and specific language will be used to describe the same. It will nevertheless be understood that no limitation of the scope of the disclosure is intended. Any alterations and further modifications to the described devices, instruments, methods, and any further application of the principles of the present disclosure are fully contemplated as would normally occur to one skilled in the art to which the disclosure relates. In particular, it is fully contemplated that the features, components, and/or steps described with respect to one implementation may be combined with the features, components, and/or steps described with respect to other implementations of the present disclosure.

The present disclosure relates to ophthalmic surgery, and more specifically, to intraocular lens (IOL) compressors adapted for use with an IOL injector and methods of compressing an IOL prior to injection of the IOL into an eye of a patient.

Following removal of a cataractous lens by phacoemulsification or by other surgical procedures, the cataractous lens is replaced by an artificial lens, referred to herein as an IOL. The IOL is typically injected into the eye through the same small incision used to remove the diseased lens. An IOL injector is used to deliver an IOL into the eye.

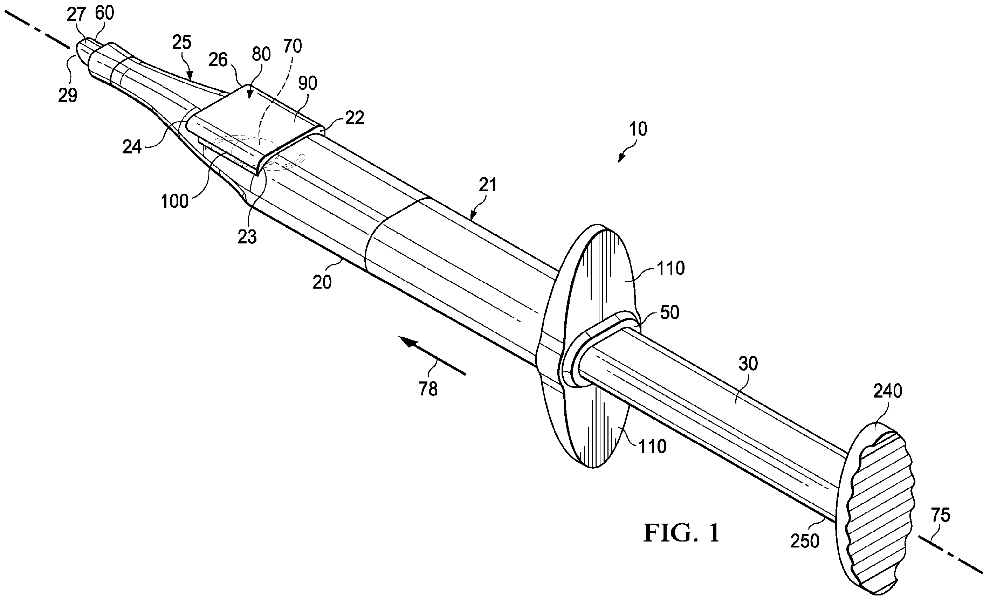

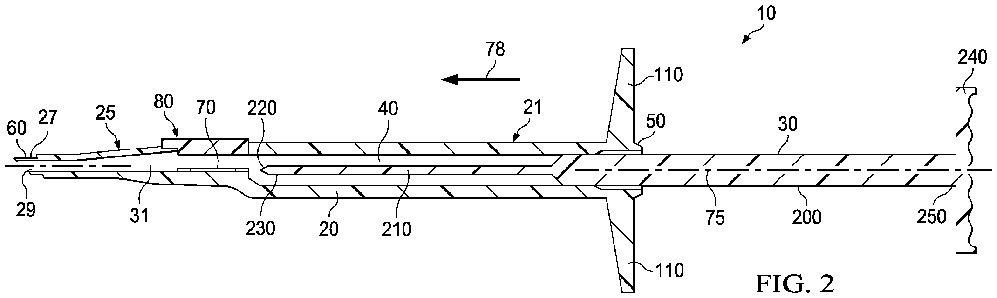

FIGs. 1 and 2 are schematics of an exemplary IOL injector 10. The IOL injector 10 has an injector body 20. The injector body 20 includes a main body 21 having a proximal end 50 and a distal end 22. The injector body 20 includes an IOL storage compartment 80 operable to house an IOL 70 prior to insertion into an eye. The IOL storage compartment 80 has a proximal end 23 and a distal end 24, the proximal end 23 of the IOL storage compartment 80 coupled to the distal end 22 of the main body 21. In some instances, a door 90 may be included to provide access to the IOL storage compartment 80. The door 90 may include a hinge 100 such that the door 90 may be pivoted about the hinge 100 to open the IOL storage compartment 80. The injector body 20 includes an injector nozzle 25 having a proximal end 26 and a distal end 60. The nozzle 25 defines a passage 31. The proximal end 26 of the nozzle 25 is coupled to the distal end of the IOL storage compartment 80. The nozzle 25 also includes a distal tip 27 that defines an opening 29 through which the IOL is delivered out of the IOL injector 10. The injector body 20 defines a bore 40 that joins and is fluid communication with the opening 29. A longitudinal axis 75 extends along the bore 40. The injector body 20 may also include tabs 110, for example formed at the proximal end 50 of the main body 21. Other configurations are possible. For example, in other implementations, the tabs 110 may be located at the distal end 22 of the main body 21. The tabs 110 may be manipulated by fingers of a user, such as an ophthalmologist or other medical professional, to advance the plunger 30 through the bore 40.

In some implementations, various manipulations of the IOL injector 10, and various method steps, may be performed by one person, or by a plurality of persons. For example, some steps of methods described herein may be performed by a nurse, while other steps may be performed by an ophthalmic surgeon. For example, compression of an IOL 70 may be performed by a nurse, while injection of the IOL 70 into an eye may be performed by a surgeon.

The IOL injector 10 also includes a plunger 30 received within the bore 40 and moveable therein such that the plunger 30 is slideable within the bore 40. As the plunger 30 is displaced distally within bore 40, the plunger 30 engages and advances an IOL, such as IOL 70, contained in the compartment 80.

As shown in FIG. 2, the plunger 30 includes a body portion 200, a plunger rod 210 extending distally from the body portion 200, and a plunger tip 220 formed at a distal end 230 of the plunger rod 210 and adapted to contact an IOL disposed, for example, within the IOL storage compartment 80 of the IOL injector 10. The plunger 30 may also include a flange 240 formed at a proximal end 250 of the body portion 200. The plunger 30 is movable along the longitudinal axis 75 within the bore 40 in response to an axial force applied to the plunger 30 in the direction of arrow 78. The axial force may be applied to the flange 240, such as by a thumb of a user.

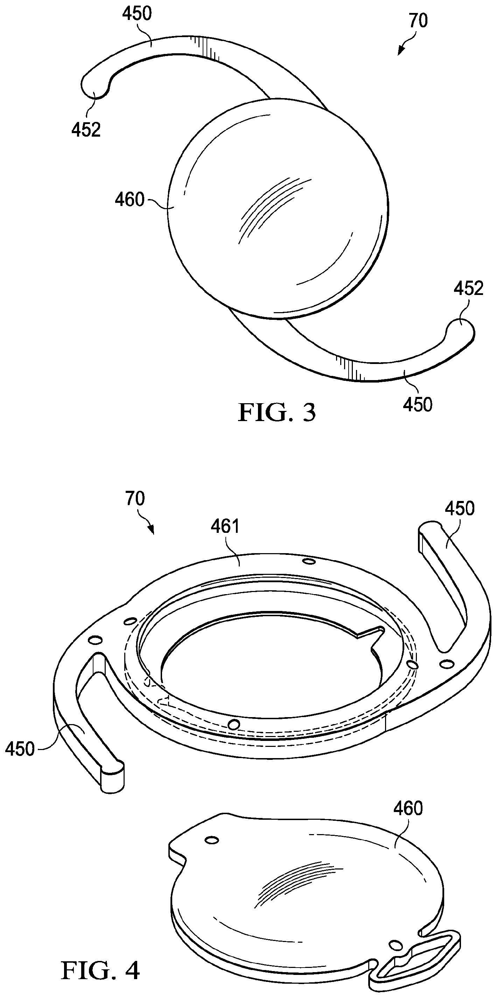

In some implementations, the IOL 70 may be a one-piece IOL. That is, in some implementations, the IOL 70 may include an optic 460 and haptics 450, as shown in FIG. 3. Each of the haptics 450 include a tip 452. In some implementations, the optic 460 and the haptics 450 may be integrally formed out of a single piece of material. In other implementations, the optic 460 may be formed out of one piece of material; the haptics 450 may be formed out of another piece of material, and the optic 460; and the haptics 450 may be coupled together prior to delivery into an eye. In some instances, the optic 460 and haptics 450 may be fixedly secured to each other prior to insertion into an IOL injector and delivered into an eye.

In other implementations, the IOL 70 may be a multi-piece IOL. For example, in some implementations, the IOL 70 be include two or more separate components. FIG. 4 is an example IOL 70 that includes two removably attached components. As shown in FIG. 4, the IOL 70 includes an optic 460 and a base 461 that includes haptics 450. The optic 460 and the base 461 are adapted to be coupled together into a unitary IOL and, thereafter, detached from each other into separate components, if desired. In some instances, one or more components of a multi-piece IOL, such as, for example the two-piece IOL 70 shown in FIG. 4, are separately injectable into a patient's eye. Once in the eye, the components may be assembled into a complete IOL. For example, the two-piece IOL 70 shown in FIG. 4, the optic 460 and the base 461 are separately injectable into an eye. Once injected, the optic 460 is adapted to be coupled to and to rest on the base 461.

Occasionally, patients may require replacement of an IOL, and a procedure to replace an IOL may result in damage to the eye. With the use of a two-piece IOL, for example, a replacement procedure may involve replacement only of the optic, allowing the base to remain in place within the eye.

As explained above, in some implementations, the IOL 70 may be a two-piece IOL wherein the base 461 and the optic 460 are separately injected into the patient's eye. Accordingly, for two-piece IOLs, the base 461 and the optic 460 may be contained in separate IOL injectors 10 for insertion in the eye. In other implementations, the two components of a two-piece IOL may be inserted into an eye separately using a single IOL injector. For a single piece IOL, the optic 460 and haptics 450 form a unitary IOL and are inserted into an eye simultaneously with the use of a single IOL injector.

Accordingly, in some implementations, a user may place a one-piece IOL into an IOL injector, for example, by loading an IOL into the IOL storage compartment of the IOL injector, such as the IOL storage compartment 80 of the IOL injector described above. As also explained, the storage compartment may be accessed via a door, such as the door 90. In some implementations, the IOL may be manually folded into a compressed or folded configuration.

In the case of a two-piece IOL, in some implementations, a user may load the base (which may be similar to base 461) into an IOL storage compartment of an IOL injector, for example, via a door. The optic (which may be similar to optic 460) of may be introduced into the IOL storage compartment of separate IOL injector, for example, via a door. In some instances, the IOL storage compartment may be accessed through the door similar to door 90. In some implementations, one or both of the base and the optic may be manually folded into a compressed or folded configuration.

In some implementations, the IOL may be pre-loaded into the storage compartment of an IOL injector, for example, during manufacturing or otherwise prior to distribution to an end user. Accordingly, for the one-piece IOL, the one-piece IOL may be pre-loaded into the storage compartment an IOL injector prior to receipt by the end user. For a two-piece IOL, the base may be pre-loaded into a storage compartment of one IOL injector, while the optic may be pre-loaded into the IOL storage compartment of another IOL injector. The term "pre-loaded" as used herein means that an IOL, either in a one-piece or multi-piece configuration (including, for example, a two-piece configuration) is loaded into the IOL injector not by a user, but, rather, the IOL is installed in the IOL injector before and is already contained within the IOL injector when the IOL injector is received by the user. The IOL injector(s) may be packaged within sterile packaging when received by a user.

As would be understood by persons of ordinary skill in the art, an IOL that is pre-loaded into an IOL injector has advantages over manual installation and folding of an IOL into the IOL injector that is performed by a user. For example, manual installation and folding of an IOL may allow more opportunity for errors, which have the potential to cause unnecessary secondary manipulation or correction during an already complex procedure. Manual installation and folding of an IOL may also introduce the possibility of contamination of the IOL, such as by human error or poor sterile technique. Contamination of the IOL may compromise the sterile environment for the patient and risk infection or other harm to the patient.

As would be understood by skilled persons, it is important for IOLs to be stored unfolded so as not to become permanently deformed over time. Accordingly, IOLs are not held in a folded condition over a long period of time e.g., in storage.

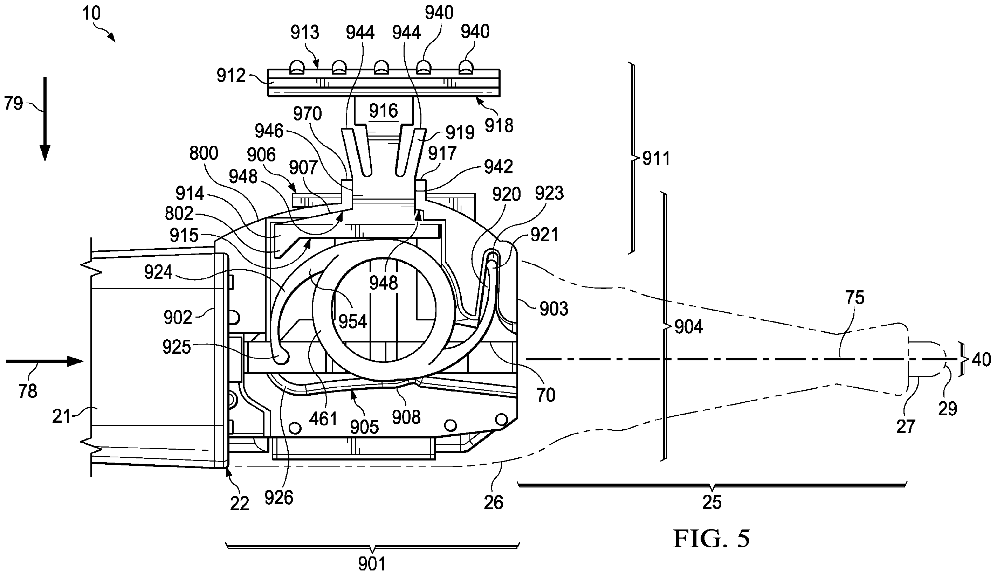

FIGs. 5-7 show an IOL compressor 901 that may be incorporated into an IOL injector. In some instances, the IOL compressor 901 may form an integral part of the IOL injector. In other instances, the IOL compressor 901 may be a separate component, such as a detachable component that may be removably connected to an IOL injector.

In the example shown in FIG. 5, the IOL compressor 901 forms an integral part of an IOL injector 10. The IOL compressor 901 includes a housing 800 and a compression chamber 904 formed within the housing 800. When the IOL compressor 901 is in an unactuated condition, the compression chamber 904 serves as a storage compartment in which an IOL (or component thereof) resides in an unstressed condition. The IOL compressor 901 may be used compress a one-piece IOL or one or more components of a multi-piece IOL. The IOL (or one or more components thereof) may be manually loaded into the compression chamber 904 of the compressor 901 by a user or the IOL (or one or more components thereof) may be pre-loaded during manufacturing or at some other time prior to delivery to a user.

In the example shown in FIGs 5-7, the IOL compressor 901 includes a proximal end 902 and a distal end 903. The proximal end 902 of the IOL compressor 901 is coupled to a distal end 22 of a main body 21 of the IOL injector 10, and the distal end 903 of the IOL compressor 901 is coupled to a proximal end 26 of a nozzle 25 of the IOL injector 10.

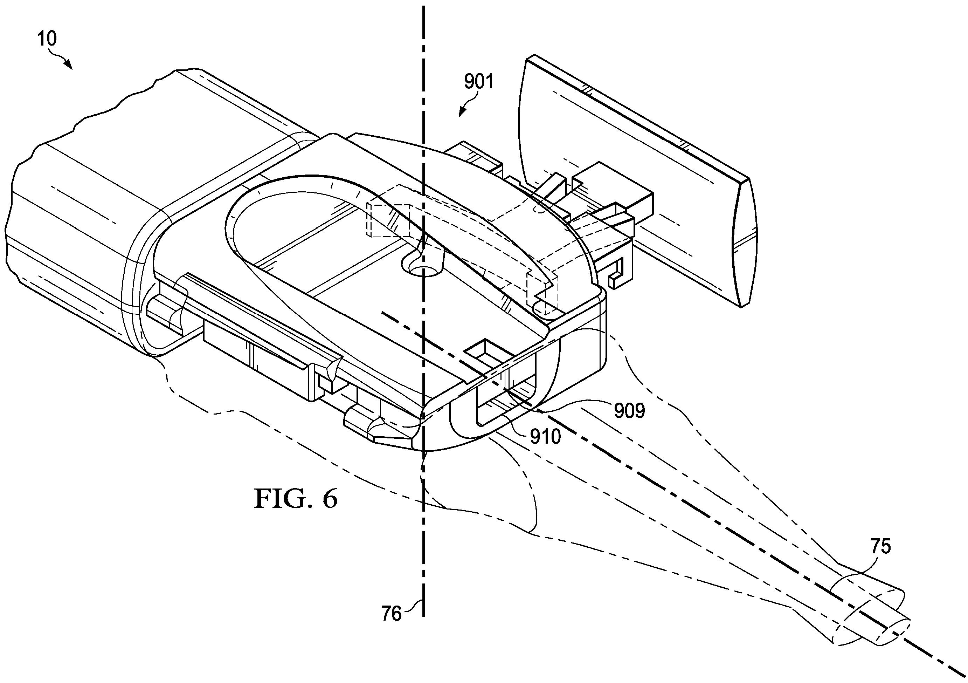

As indicated above, the IOL compressor 901 includes the compression chamber 904 that is adapted to receive an IOL or component thereof (hereinafter collectively referred to as "IOL"). The compression chamber 904 is adapted to house the IOL 70 in an unfolded state before compression of the IOL 70 occurs. The compression chamber 904 includes an interior surface 905 adapted to contact the IOL 70, an exterior surface 906, a first side 907, and a second side 908. A distance between the first side 907 and the second side 908 defines an interior width of the compression chamber 904. The interior width may be typically configured to accommodate the transverse width of an unfolded IOL. Referring to FIG. 6, the IOL compression chamber 904 also includes a third side 909 and fourth side 910. A distance between the third side 909 and the fourth side 910 defines an interior height of the compression chamber 904. Referring again to FIG. 5, a distance between the proximal end 902 of the IOL compressor 901 and the distal end 903 of the IOL compressor 901 defines a length of the compression chamber 904. A plunger (which may be similar to plunger 30 described above) is slideable within a bore defined by the main body 21 of the IOL injector 10 (which may be similar to the bore 40 described above). As the plunger is advanced, a plunger rod of the plunger passes through the compression chamber 904 from the proximal end 50 of the main body 21, and, as the plunger continues to advance, the plunger rod is made to pass through the distal tip 27 and, ultimately, an opening 29 formed in the nozzle 25. Accordingly, the IOL compression chamber 904 is coupled with the main body 21 and the nozzle 25 such that the bore defined by the main body 21, the compression chamber defined by the IOL compressor 901, and a passage defined by the nozzle 25 (which may be similar to the passage 31, described above) are aligned and communicate with each other, allowing passage of the injector rod of the plunger through the main body 21, the compression chamber 904, and the nozzle 25.

The IOL compressor 901 also includes a transversely depressible button 911. The button 911 includes a pad 912 located outside the compression chamber 904; an internal portion 914 located inside of the compression chamber 904 and defining a compression surface 915; and a stem 916 connecting the pad 912 and the internal portion 914. The pad 912 includes a contact surface 913 accessible to a user. In some implementations, the contact surface 913 may have surface features, such as raised dots 940, to define a textured surface, that provides grip for the user's finger on the contact surface 913. The pad 912 is adapted to be depressible by a user, e.g., by a finger of the user. When depressed, the button moves into and transversely across the compression chamber 904 in the direction of arrow 79. The internal portion 914 includes a protrusion 802 located at a proximal end of the internal portion 914 and extending inwardly towards the longitudinal axis 75.

The compression surface 915 of the internal portion 914 is adapted to contact the IOL, such as, for example, a base of a two-piece IOL (which may be similar to the base 461 shown in FIG. 5). The compression surface 915 has a length. The length of the compression surface 915 may conform to a dimension of the IOL 70 in either a compressed or uncompressed condition. As the button 911 is depressed, the contact surface 915 compresses the IOL 70 between the compression surface 915 and the internal surface 905.

The stem 916 is slideably disposed within a slot 942 formed in the housing 800 of the compression chamber 904 and which defines an opening 970 in the housing 800. The slot 942 is adapted to allow smooth movement of the button 911 as the button 911 is depressed. As shown in FIG. 5, the stem 916 includes flexible wings 919. As the button 911 is depressed, the wings 919 fold inwardly towards each other as the stem 916 passes through the slot 942. When ends 944 of the wings 919 advance past an end 946 of the slot 942 interior of the compression chamber 904, the wings 919 expand, locking the depressed button 911 into position. With the wings 919 expanded, the ends 944 contact interior surfaces 948 of the compression chamber 904, thereby capturing the button 911 and preventing withdrawal thereof from the compression chamber 904, i.e., movement of the button 911 in a direction opposite that of arrow 79. With the button 911 prevented from being withdrawn from the compression chamber 904, the internal portion 914 of the button maintains the IOL 70 in a compressed state. In other implementations, the wings 919 may be omitted. In some implementations, the wings 919 may be formed of a flexible material, such as a bendable plastic material. With the button 911 locked into a depressed state by the wings 919, a user is able to release the button 911, freeing up a hand, and allowing the user to focus on delivering the IOL 70 into an eye.

In some implementations, the IOL compressor 901 may include a door, similar to the door 90 as shown in FIG. 1, to provide access to introduce the IOL disposed in the IOL compression chamber 904. In other implementations, the compressor 901 may omit a door.

In some implementations, the exterior surface 906 of the housing 800 of the compression chamber 904 may include a seat 917, and the pad 912 may include a seating surface 918 opposite the contact surface 913. The seating surface 918 is operable to contact with the seat 917 of the compression chamber 904 to limit an amount by which the button 911 may be depressed by a user. A distance between the seating surface 918 and the seat 917 when the button 911 is in an initial, unactuated position (as shown in FIG. 5) defines an amount by which the button 911 may be depressed when actuated. This distance may be selected to allow a user to compress an IOL into a compressed state having a selected, predetermined width.

### In

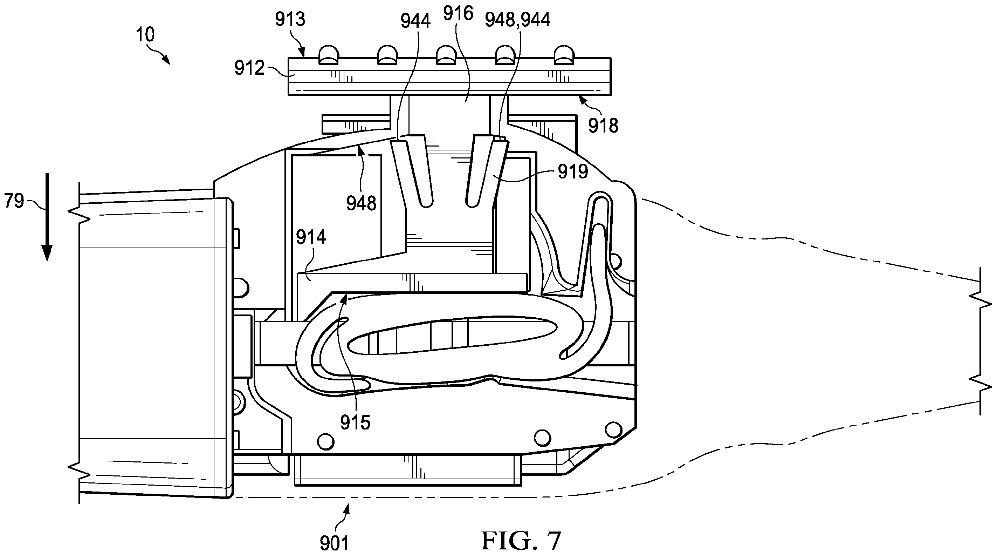

Referring to FIG. 7 The button 911 in shown in a fully transversely depressed configuration. In this configuration, the seating surface 918 of the button 911 is in contact with the seat 917 of the compression chamber 901, and ends 944 of the wings 919 are engaged with the interior surface 948 of the compression chamber 904.

Referring to FIG. 5, the IOL 70 may be a base of a multi-piece IOL (e.g., a two-piece IOL) and may include haptics. For example, the base may be similar to base 461, and the haptics may be similar to haptic 450, shown in FIG. 4. In other instances, the IOL 70 may be a single-piece IOL, similar to the IOL 70 shown in FIG. 3. The single-piece IOL may include an optic and haptics, which may be similar to the optic 460 and haptics 450 show in FIG. 4.

FIG. 5 further shows the IOL 70 is disposed in the compression chamber 904. As shown, the IOL 70 includes a leading haptic 920 having a tip 921 and the leading haptic 920 disposed in a slot 923 formed in the compression chamber 904. The slot 923 is adapted to receive the leading haptic 920 and temporarily restrain the leading haptic 920 as the IOL 70 is advanced toward the distal end 903 of the compression chamber 904. The term "leading haptic" as used herein refers to a haptic of an IOL or IOL that is more distally positioned and located adjacent to the distal end 903 of the compression chamber 904, as contrasted with a trailing haptic. A trailing haptic, as used herein, refers to a haptic of an IOL or IOL that is more proximally positioned and located adjacent to the proximal end 902 of the compression chamber 904.

Upon compression of the IOL 70 by the button 911, the slot 923 maintains a position and orientation of the leading haptic 920. While the IOL 70 remains compressed, the slot 923 substantially maintains a longitudinal position of the tip 921 of the leading haptic 920 as the IOL 70 is subsequent advanced towards the distal end 903 in response to a longitudinal axial force applied to the compressed IOL 70 by the plunger in the direction of arrow 78. The position of the tip 921 of the leading haptic 920 is substantially maintained because, as shown in FIGs. 5 and 7, the slot 923 defines an oblique angle relative to the longitudinal axis 75. Thus, as the compressed IOL 70 is advanced in the direction of arrow 78, a position of the tip 921 of the leading haptic 920 continues to move along the slot 923 inwardly towards the base 461 and longitudinal axis 75. Because of the oblique angle of the slot 923, as the tip 921 moves inwardly, the longitudinal position of the tip 921 moves slightly proximally. An angle of the slot 923 relative to the longitudinal axis 75 may be selected such that, when the IOL 70 is loaded into the compression chamber 904, the slot 923 corresponds to an unstressed shape of the leading haptic 920. In other implementations, the angle of the slot 923 relative to the longitudinal axis 75 may be any desired angle.

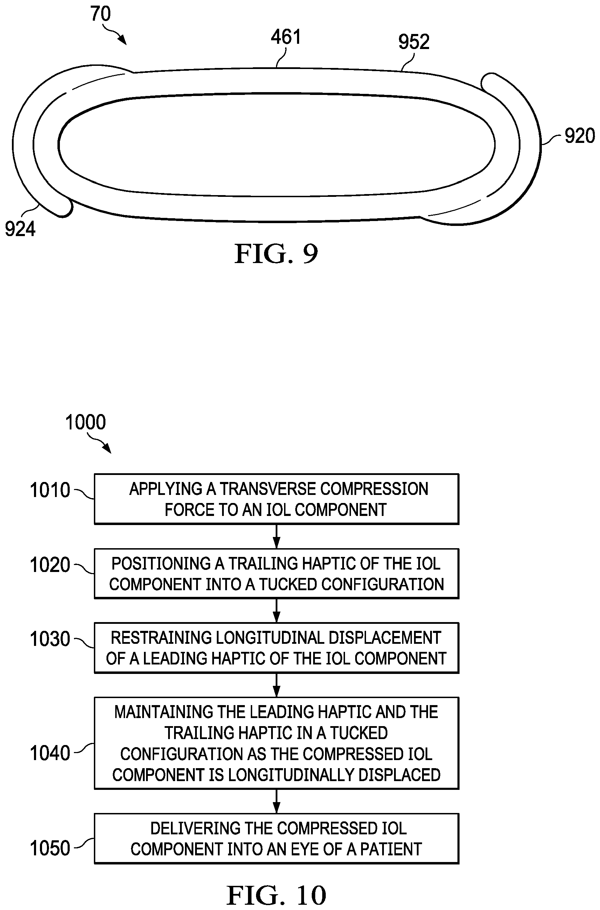

By containing the leading haptic 920 within the slot 923 as the compressed IOL 70 is advanced by the plunger, the leading haptic 920 is prevented from extending distally beyond a distal end of the compressed base or optic of the IOL 70. The slot 923 guides the leading haptic 920 and controls how the leading haptic 920 folds relative to the base or optic of the IOL 70. As the IOL 70 is advanced by the plunger, the interaction between the leading haptic 920 and the slot 921 results in the leading haptic 920 being folded about the optic or base of the IOL 70 and abutting an outer surface of the base or optic of the IOL 70. FIG. 9 shows an example IOL 70 in the form of a base of a two-piece IOL. The example IOL 70 includes a base 461, a leading haptic 920 and a trailing haptic 924. As shown in FIG. 9, once the IOL 70 is advanced sufficiently such that the leading haptic 920 is removed from the slot 923, the leading haptic 920 conforms to an exterior perimeter 952 of the base 461.

By avoiding an untuck configuration of the leading haptic 920, i.e., a configuration in which the leading haptic 920 does not abut and conform to the exterior perimeter 952 of the base 461, a surgeon avoids additional interaction with the IOL 70 in an effort to insert successfully the IOL into a patient's eye, thereby reducing the time required for the surgical procedures; reducing the risk of injury to the patient's eye; and reducing or eliminating the risk of damage to the leading haptic 920 or other part of the IOL 70. With reduced risk of damage to the IOL 70, a surgeon also reduces or eliminates the need to remove and replace a damaged IOL 70The leading haptic 920 maintains this orientation relative to the base 461 as the IOL 70 advances axially through the IOL injector 10 and is expelled from the opening 29 of the nozzle 25 of the IOL injector 10.

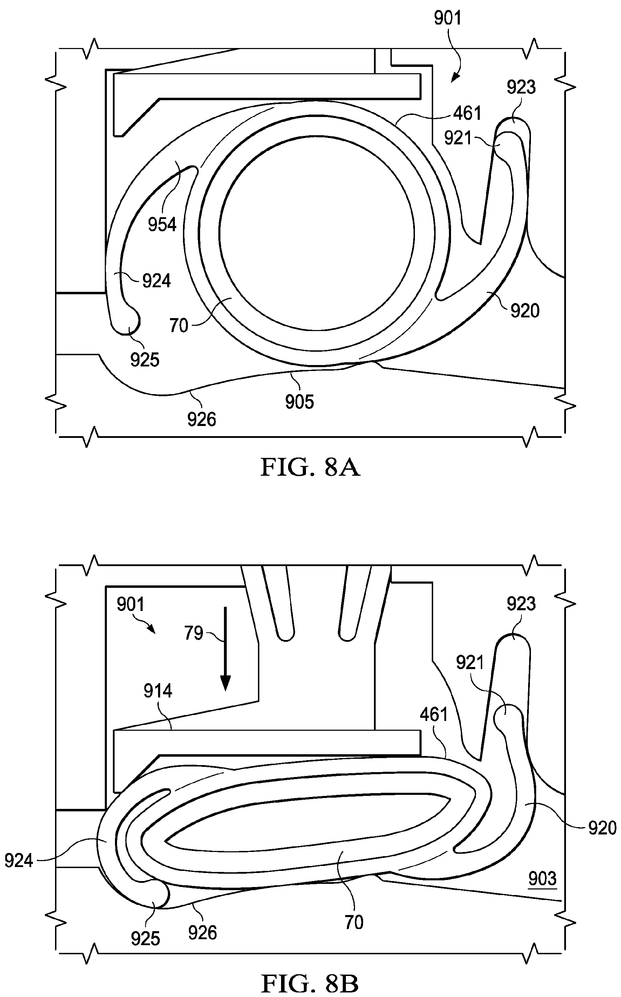

Referring again to FIGs. 5 and 7-9, as the button 911 is depress and the interior portion 914 of the button 911 compresses the IOL 70, a tip 925 of the trailing haptic 924 is displaced towards a curved portion 926 of the interior surface 905. The protrusion 802 of the internal portion 914 is adapted to contact the trailing haptic 924 and prevent the trailing haptic 924 from moving proximally as the IOL 70 is compressed. As IOL 70 continues to be compressed, the tip 925 engages the curved portion 926, and the tip 925 travels along the curved portion 926, causing the trailing haptic 924 to pivot about a shoulder 954 where the trailing haptic 924 joins the base 461. By following the curved portion 926, the tip 925 of the trailing haptic 924 moves distally, ultimately resulting in the trailing haptic 924 taking on a tucked configuration, i.e., abutting and conforming to the exterior perimeter 952 of the base 461, as shown in FIG. 8B. Thus, the curved portion 926 of the interior surface 905 controls the orientation of the trailing haptic 924 as the IOL 70 is compressed by the button 911. The protrusion 802 operates to maintain the trailing haptic 924 in the tucked configuration, Thus, when the IOL 70 is fully compressed by the button 911, the trailing haptic 924 is tucked close to the base 461.

With the trailing haptic 924 oriented as described above, the risk of the trailing haptic 924 being displaced from an abutting relationship with the base 961 and trailing behind the tip of the plunger (i.e., being disposed proximally relative to the tip of the plunger) as the plunger advances the compressed IOL 70 through the IOL injector 10 is eliminated or substantially reduced. As a result, a surgeon avoids additional interaction with the IOL 70 in an effort to insert successfully the IOL 70 into a patient's eye, reducing the time required for the surgical procedures; reducing the risk of injury to the patient's eye; and reducing or eliminating the risk of damage to the trailing haptic. With reduced risk of damage to the IOL, a surgeon also reduces or eliminates the need to remove and replace a damaged IOL.

FIGs. 8A and 8B show an IOL 70 (in this instance, a base of a two-piece IOL) in an uncompressed state and a compressed state, respectively. As shown in FIG. 8A, the tip 921 of the leading haptic 920 is disposed within the slot 923. The tip 925 of the trailing haptic 924 is shown adjacent to a proximal end of the curved portion 926 of the interior surface 905. As shown in FIG. 8B, the IOL 70 is in a compressed stated with the tip 921 still located within the slot 923 and the trailing haptic 924 abutted against and conforming to the compressed shape of the base 461. In FIG. 8B, the internal portion 914 of the button 911 is shown in contact with the IOL 70, transversely compressing the IOL 70.

Compression of the IOL 70 shown in FIG. 5, 7, 8A, 8B, and 9 (i.e., a base of a two-piece IOL) results in the IOL 70 maintaining a height dimension that is essentially unchanged. That is, while the IOL 70 is compressed laterally, the IOL 70 is prevented from bowing or otherwise becoming distorted in a direction defined by vertical axis 76. Thus, the compression chamber 904 operates to maintain the compressed IOL 70 in an essentially planar configuration between the third side 909 and the fourth side 910 of the compression chamber 904, shown in FIG. 6.

The present disclosure also relates to methods of compressing an IOL or component thereof. An example method 1000 is shown in FIG. 10. At 1010, the method 1000 includes applying a transverse compression force to an IOL. With application of the transverse compression force to the IOL, a base of the IOL is placed into a compressed state. At 1020, a trailing haptic of the IOL is placed into a tucked configuration in which the trailing haptic is made to abut against and conform to a shape of a base of the IOL. With the base compressed by the transverse compression force, the trailing haptic abuts and conforms to the base in the compressed state. At 1030, a longitudinal displacement of leading haptic of the IOL is restrained so as to maintain a longitudinal position of the leading haptic relative to the base. The longitudinal position of the leading haptic may be restrained by defining a path along which the leading haptic is permitted to travel as the IOL is compressed and longitudinally displaced. At 1040, both the leading haptic and trailing haptic are maintained in a tucked configuration as the compressed IOL is longitudinally displaced. At 1050, the compressed IOL is delivered into an eye of a patient.

Other methods may include additional, fewer, or different steps. For example, in other implementations, the IOL may be contained in an IOL compression chamber (which may be similar to IOL compression chamber 904, described above) when the IOL is compressed. The transverse compression force may be applied to the IOL by a button (which may be similar to button 911, described above). The button may be displaced transversely relative to a longitudinal axis of the compression chamber, thereby decreasing a transverse width of the IOL. In some implementations, the button may include a stem that slideable within a slot formed in the compression chamber. In some implementations, the button 911 may also include an internal portion having a compression surface operable to compress the IOL between the compression surface and an interior surface of compression chamber. In some implementations, depression of the button may be limited by contact between a portion of the button and a surface of the compression chamber, such as, for example, an exterior surface..

In some implementations, the method 1000 may include locking the IOL compression chamber in a compressed configuration. For example, locking the IOL compression chamber in a compressed configuration may include preventing withdrawal of the button from a fully depressed position. Preventing withdrawal of the button from a fully depressed position may include generating interference between flexible wings of the button and a surface of the compression chamber, such as, for example, an interior of the compression chamber.

The transverse compression force applied in the direction of arrow 79 (shown, for example, in FIG. 5) is typically perpendicular or approximately perpendicular to the axial force applied to the IOL by the plunger in the direction of arrow 78 (also shown in FIG. 5). In some implementations, the transverse compression force may be applied prior to the IOL application of the longitudinal axial force by the plunger.

In some implementations, the application of the transverse compression force may decrease a transverse width of the IOL. In some instances, compression of the IOL may reduce a width of the IOL to less than 50% of the uncompressed width of the IOL. The uncompressed width of the IOL may be a width dimension in a direction perpendicular to a longitudinal axis of an IOL injector. In some implementations, compression of the IOL results in changing a shape of the IOL from an approximately circular shape into an approximately oval shape, as shown, for example, in Figure 8.

Application of a transverse compression force to the IOL may decrease the risk of buckling of the IOL upon the application of the longitudinal axial force to the IOL by a plunger. Buckling may occur as a result of axial loading of an IOL by a plunger when the IOL is in an uncompressed condition. Accordingly, compression applied to an IOL, e.g., by a compressor similar to compressor 901, described above, provides increased stability when applying axial loading to the compressed IOL along a longitudinal axis of an IOL injector by a plunger during delivery of the IOL into an eye of a patient. Consequently, compressing an IOL prior to axial loading by a plunger decreases the possibility that the axial loading will result in buckling of the IOL.

Various implementations of an IOL compressor and methods of compressing an IOL described herein and within the scope of the present disclosure may be used in or otherwise applicable to an IOL injector configured to inject an IOL base and/or an IOL optic of a multi-piece IOL. Various implementations of an IOL compressor and associated methods of compressing an IOL described herein may be used with an IOL base and/or the IOL optic that are manually loaded into a compression chamber by a user or preloaded thereinto prior to delivery to a user.

Non-limiting examples of IOL injectors that may be adapted for use with the IOL compressor as described herein include those described in U.S. Patent No. 7,156,854 and U.S. Patent Application Publication No. 2016/0256316.

Advantages of the IOL compressors s described herein include the ability easily and reliably to compress a single-piece IOL or the separate components of a multi-piece IOL into a compressed state prior to advancement by a plunger and delivery into an eye..

The above-disclosed subject matter is to be considered illustrative, and not restrictive, and the appended claims are intended to cover all such modifications, enhancements, and other implementations that fall within the scope of the present disclosure. Thus, to the maximum extent allowed by law, the scope of the present disclosure is to be determined by the broadest permissible interpretation of the following claims and their equivalents and shall not be restricted or limited by the foregoing detailed description.

## Claims

1. An intraocular lens (70) injector (10) comprising:
an injector body (20) defining a bore (40);
a nozzle (25) coupled to the injector body, the nozzle comprising an opening (29) in fluid communication with the bore;
a plunger (30) receivable into the bore and moveable therein; and
an IOL compressor (901) coupled to the injector body, the IOL compressor comprising:
a housing (800);
a compression chamber (904) formed within the housing and adapted to receive an IOL;
a transversely depressible button (911) extending through a slot (942) formed in the housing, the transversely depressible button moveable within the slot, the transversely depressible button comprising:
a pad (912) located outside of the housing of the compression chamber;
an internal portion (914) located inside of the compression chamber, the internal portion having a compression surface (915) adapted to compress the IOL disposed in the compression chamber;
a stem (916) connecting the pad and the internal portion, the stem slideably disposed within the slot; and
at least one flexible wing (919) extending from the stem, the at least one wing is adapted to flex inwardly when passing through the slot and to expand outwardly when the at least one flexible wing is received into the compression chamber;
wherein the compression chamber defines an interior surface (948), and wherein the at least one flexible wing cooperates with the interior surface to form a lock that is adapted to hold the transversely depressible button in a depressed state when the at least one flexible wing is received in the compression chamber.

2. The IOL injector (10) of claim 1 wherein the housing (800) of the compressor comprises an exterior surface that defines a seat (917),
wherein the pad (912) of the transversely depressible button comprises a seating surface (918), and
wherein the seating surface is adapted to contact the seat to stop movement of the transversely depressible button (911) in a first transverse direction.

3. The IOL injector (10) of claim 1 wherein the compression chamber (904) defines a slot (923) adapted to receive a leading haptic (920) of the IOL (70) and to restrain longitudinal movement of the leading haptic as the IOL is compressed within the compression chamber.

4. The IOL injector (10) of claim 1 wherein the compression chamber comprises (904) an interior surface (905, 948) that includes a curved portion (926) adapted to guide a trailing haptic (924) of the IOL (70) into a tucked configuration as the IOL is compressed within the compression chamber.

5. A method (1000) of compressing an intraocular lens (70) in the intraocular lens injector according to claims 1 to 4, the method comprising:
applying (1010) a transverse compression force to the intraocular lens, the intraocular lens comprising a base (461), a trailing haptic (924) coupled to the base, and a leading haptic (920) coupled to the base;
positioning (1020) the trailing haptic of the intraocular lens into a tucked configuration as the intraocular lens is compressed;
restraining (1030) longitudinal displacement of the leading haptic as the intraocular lens is compressed;
positioning (1040) the leading haptic into a tucked configuration as the compressed intraocular lens is longitudinally displaced; and
maintaining (1040) both the leading haptic and the trailing haptic in a tucked configuration as the compressed intraocular lens continues to be longitudinally displaced.

6. The method (1000) of claim 5, wherein applying (1010) a transverse compression force to the intraocular lens (70) comprises depressing a transversely depressible button (911) to compress the intraocular lens between the button and an interior surface (948) of an intraocular lens compressor (901).

7. The method (1000) of claim 5, wherein positioning (1040) the trailing haptic (924) of the intraocular lens (70) into a tucked configuration as the intraocular lens is compressed comprises engaging a tip (925) of the trailing haptic with a curved surface (926) to guide the trailing haptic into an abutting and conforming relationship with the base (461) of the intraocular lens.

8. The method (1000) of claim 5, wherein restraining (1030) longitudinal displacement of the leading haptic (920) as the intraocular lens (70) is compressed comprises positioning the leading haptic in a slot (942) that is adapted to define a path of travel of the leading haptic as the intraocular lens is compressed.

9. The method (1000) of claim 6, wherein positioning (1040) the leading haptic (920) into a tucked configuration as the compressed intraocular lens (70) is longitudinally displaced comprises longitudinally displacing the compressed intraocular lens while restraining longitudinal movement of the leading haptic such that the leading haptic abuts and conforms to a shape of the base (461).

## Patentansprüche

1. Injektor (10) für Intraokularlinsen (70), umfassend:
einen Injektorkörper (20), der eine Bohrung (40) definiert,
eine Düse (25), die an den Injektorkörper gekoppelt ist, wobei die Düse eine Öffnung (29) in Fluidverbindung mit der Bohrung umfasst,
einen Kolben (30), der in der Bohrung aufnehmbar und darin beweglich ist, und
einen IOL-Kompressor (901), der an den Injektorkörper gekoppelt ist, wobei der IOL-Kompressor Folgendes umfasst:
ein Gehäuse(800),
eine Kompressionskammer (904), die in dem Gehäuse ausgebildet und zur Aufnahme einer IOL ausgeführt ist,
einen quer niederdrückbaren Knopf (911), der sich durch einen in dem Gehäuse ausgebildeten Schlitz (942) erstreckt, wobei der quer niederdrückbare Knopf in dem Schlitz beweglich ist, wobei der quer niederdrückbare Knopf Folgendes umfasst:
eine Unterlage (912), die sich außerhalb des Gehäuses der Kompressionskammer befindet,
einen inneren Abschnitt (914), der sich in der Kompressionskammer befindet, wobei der innere Abschnitt eine Kompressionsfläche (915) aufweist, die zum Komprimieren der in der Kompressionskammer angeordneten IOL ausgeführt ist,
einen Schaft (916), der die Unterlage und den inneren Abschnitt verbindet, wobei der Schaft verschiebbar in dem Schlitz angeordnet ist, und
mindestens einen flexiblen Flügel (919), der sich von dem Schaft erstreckt, wobei der mindestens eine Flügel dazu ausgeführt ist, sich nach innen zu biegen, wenn er durch den Schlitz hindurchgeht, und nach außen zu expandieren, wenn der mindestens eine flexible Flügel in der Kompressionskammer aufgenommen wird,
wobei die Kompressionskammer eine Innenfläche (948) definiert und wobei der mindestens eine flexible Flügel mit der Innenfläche zusammenwirkt, um eine Verriegelung zu bilden, die dazu ausgeführt ist, den quer niederdrückbaren Knopf in einem niedergedrückten Zustand zu halten, wenn der mindestens eine flexible Flügel in der Kompressionskammer aufgenommen ist.

2. IOL-Injektor (10) nach Anspruch 1, wobei das Gehäuse (800) des Kompressors eine Außenfläche umfasst, die einen Sitz (917) definiert,
wobei die Unterlage (912) des quer niederdrückbaren Knopfs eine Sitzfläche (918) umfasst und
wobei die Sitzfläche zum Kontaktieren des Sitzes ausgeführt ist, um die Bewegung des quer niederdrückbaren Knopfs (911) in einer ersten Querrichtung zu stoppen.

3. IOL-Injektor (10) nach Anspruch 1, wobei die Kompressionskammer (904) einen Schlitz (923) definiert, der zur Aufnahme einer vorderen Haptik (920) der IOL (70) und zur Beschränkung einer Längsbewegung der vorderen Haptik ausgeführt ist, wenn die IOL in der Kompressionskammer komprimiert wird.

4. IOL-Injektor (10) nach Anspruch 1, wobei die Kompressionskammer (904) eine Innenfläche (905, 948) umfasst, die einen gekrümmten Abschnitt (926) aufweist, der dazu ausgeführt ist, eine hintere Haptik (924) der IOL (70) in eine gestauchte Konfiguration zu führen, wenn die IOL in der Kompressionskammer komprimiert wird.

5. Verfahren (1000) zum Komprimieren einer Intraokularlinse (70) in dem Intraokularlinseninjektor nach Ansprüchen 1 bis 4, wobei das Verfahren Folgendes umfasst:
Aufbringen (1010) einer Querkompressionskraft auf die Intraokularlinse, wobei die Intraokularlinse eine Basis (461), eine an die Basis gekoppelte hintere Haptik (924) und eine an die Basis gekoppelte vordere Haptik (920) umfasst,
Positionieren (1020) der hinteren Haptik der Intraokularlinse in eine gestauchte Konfiguration, wenn die Intraokularlinse komprimiert wird,
Einschränken (1030) der Längsverschiebung der vorderen Haptik, wenn die Intraokularlinse komprimiert wird,
Positionieren (1040) der vorderen Haptik in eine gestauchte Konfiguration, wenn die komprimierte Intraokularlinse in Längsrichtung verschoben wird, und
Beibehalten (1040) sowohl der vorderen Haptik als auch der hinteren Haptik in einer gestauchten Konfiguration, während die komprimierte Intraokularlinse weiterhin in Längsrichtung verschoben wird.

6. Verfahren (1000) nach Anspruch 5,
wobei das Aufbringen (1010) einer Querkompressionskraft auf die Intraokularlinse (70) das Niederdrücken eines quer niederdrückbaren Knopfs (911) umfasst, um die Intraokularlinse zwischen dem Knopf und einer Innenfläche (948) eines Intraokularlinsenkompressors (901) zu komprimieren.

7. Verfahren (1000) nach Anspruch 5,
wobei das Positionieren (1040) der hinteren Haptik (924) der Intraokularlinse (70) in eine gestauchte Konfiguration beim Komprimieren der Intraokularlinse das Ineingriffbringen einer Spitze (925) der hinteren Haptik mit einer gekrümmten Fläche (926) umfasst, um die hintere Haptik in eine anliegende und konforme Beziehung mit der Basis (461) der Intraokularlinse zu führen.

8. Verfahren (1000) nach Anspruch 5,
wobei das Einschränken (1030) der Längsverschiebung der vorderen Haptik (920) beim Komprimieren der Intraokularlinse (70) das Positionieren der vorderen Haptik in einen Schlitz (942) umfasst, der dazu ausgeführt ist, einen Verfahrweg der vorderen Haptik zu definieren, wenn die Intraokularlinse komprimiert wird.

9. Verfahren (1000) nach Anspruch 6, wobei das Positionieren (1040) der vorderen Haptik (920) in eine gestauchte Konfiguration, wenn die komprimierte Intraokularlinse (70) in Längsrichtung verschoben wird, das Längsverschieben der komprimierten Intraokularlinse umfasst, während die Längsbewegung der vorderen Haptik so eingeschränkt wird, dass die vordere Haptik an einer Form der Basis (461) anliegt und damit konform ist.

## Revendications

1. Injecteur (10) de lentille intraoculaire (70) comprenant :
un corps d'injecteur (20) définissant un orifice (40) ;
une buse (25) accouplée au corps d'injecteur, la buse comprenant une ouverture (29) en communication fluidique avec l'orifice ;
un piston (30) propre à être reçu dans l'orifice et à être déplacé dans celui-ci ; et
un compresseur de lentille intraoculaire (901) accouplé au corps d'injecteur, le compresseur de lentille intraoculaire comprenant :
un boîtier (800) ;
une chambre de compression (904) formée à l'intérieur du boîtier et propre à recevoir une lentille intraoculaire ;
un bouton (911) enfonçable transversalement s'étendant à travers une fente (942) formée dans le boîtier, le bouton enfonçable transversalement étant déplaçable à l'intérieur de la fente, le bouton enfonçable transversalement comprenant :
une plaquette (912) située à l'extérieur du boîtier de la chambre de compression ;
une partie interne (914) située à l'intérieur de la chambre de compression, la partie interne comportant une surface de compression (915) propre à comprimer la lentille intraoculaire disposée dans la chambre de compression ;
une tige (916) raccordant la plaquette et la partie interne, la tige étant disposée à coulissement à l'intérieur de la fente ; et
au moins une ailette flexible (919) s'étendant à partir de la tige, l'au moins une ailette étant propre à fléchir vers l'intérieur lors de son passage à travers la fente et à se déployer vers l'extérieur lorsque l'au moins une ailette flexible est reçue dans la chambre de compression ;
dans lequel la chambre de compression définit une surface intérieure (948), et dans lequel l'au moins une ailette flexible coopère avec la surface intérieure pour former un dispositif de blocage qui est propre à maintenir le bouton enfonçable transversalement dans un état enfoncé lorsque l'au moins une ailette flexible est reçue dans la chambre de compression.

2. Injecteur de lentille intraoculaire (10) selon la revendication 1, dans lequel le boîtier (800) du compresseur comprend une surface extérieure qui définit un siège (917),
dans lequel la plaquette (912) du bouton enfonçable transversalement comprend une surface d'appui (918), et dans lequel la surface d'appui est propre à venir en contact avec le siège afin d'arrêter le mouvement du bouton (911) enfonçable transversalement dans un premier sens transversal.

3. Injecteur de lentille intraoculaire (10) selon la revendication 1, dans lequel la chambre de compression (904) définit une fente (923) propre à recevoir un haptique antérieur (920) de la lentille intraoculaire (70) et à limiter un mouvement longitudinal de l'haptique antérieur tandis que la lentille intraoculaire est comprimée à l'intérieur de la chambre de compression.

4. Injecteur de lentille intraoculaire (10) selon la revendication 1, dans lequel la chambre de compression (904) comprend une surface intérieure (905, 948) qui comporte une partie courbe (926) propre à guider un haptique postérieur (924) de la lentille intraoculaire (70) de façon à ce qu'il adopte une configuration repliée tandis que la lentille intraoculaire est comprimée à l'intérieur de la chambre de compression.

5. Procédé (1000) de compression d'une lentille intraoculaire (70) dans l'injecteur de lentille intraoculaire selon les revendications 1 à 4, le procédé comprenant :
appliquer (1010) une force de compression transversale à la lentille intraoculaire, la lentille intraoculaire comprenant une base (461), un haptique postérieur (924) accouplé à la base et un haptique antérieur (920) accouplé à la base ;
positionner (1020) le haptique postérieur de la lentille intraoculaire dans une configuration repliée tandis que la lentille intraoculaire est comprimée ;
limiter (1030) un déplacement longitudinal du haptique antérieur tandis que la lentille intraoculaire est comprimée ;
positionner (1040) le haptique antérieur dans une configuration repliée tandis que la lentille intraoculaire comprimée est déplacée longitudinalement ; et
maintenir (1040) à la fois le haptique antérieur et le haptique postérieur dans une configuration repliée tandis que la lentille intraoculaire comprimée continue d'être déplacée longitudinalement.

6. Procédé (1000) selon la revendication 5,
dans lequel l'application (1010) d'une force de compression transversale à la lentille intraoculaire (70) comprend l'enfoncement d'un bouton (911) enfonçable transversalement afin de comprimer la lentille intraoculaire entre le bouton et une surface intérieure (948) d'un compresseur de lentille intraoculaire (901).

7. Procédé (1000) selon la revendication 5,
dans lequel le positionnement (1040) du haptique postérieur (924) de la lentille intraoculaire (70) dans une configuration repliée tandis que la lentille intraoculaire est comprimée comprend la mise en prise d'une extrémité (925) du haptique postérieur avec une surface courbe (926) afin de guider le haptique postérieur de façon à ce qu'il vienne s'accoler à la base (461) de la lentille intraoculaire et s'adapte à sa forme.

8. Procédé (1000) selon la revendication 5,
dans lequel la limitation (1030) d'un déplacement longitudinal du haptique antérieur (920) tandis que la lentille intraoculaire (70) est comprimée comprend le positionnement du haptique antérieur dans une fente (942) qui est propre à définir une trajectoire du haptique antérieur tandis que la lentille intraoculaire est comprimée.

9. Procédé (1000) selon la revendication 6, dans lequel le positionnement (1040) du haptique antérieur (920) dans une configuration repliée tandis que la lentille intraoculaire (70) comprimée est déplacée longitudinalement comprend le déplacement longitudinal de la lentille intraoculaire comprimée tout en limitant un mouvement longitudinal du haptique antérieur de telle sorte que le haptique antérieur vienne s'accoler à la base (461) et s'adapte à sa forme.
